# EUROPÄISCHE PATENTSCHRIFT

(11) **EP 0 496 274 B1**
(45) Veröffentlichungstag und Bekanntmachung des Hinweises auf die Patenterteilung: **03.07.1996**
(21) Anmeldenummer: 92100637.5
(22) Anmeldetag: 16.01.1992
(51) Int. Cl.: C07D 455/06, A61K 31/435

(54) **Tricyclisches Pyridonderivat**
Tricyclic pyridone derivative
Dérivé tricyclique de la pyridone

(30) Priorität: 25.01.1991 CH 228/91
(43) Veröffentlichungstag der Anmeldung: 29.07.1992
(73) Patentinhaber: F. Hoffmann-La Roche AG, CH-4002 Basel (CH)
(72) Erfinder: Scherschlicht, Richard Raimund, Dr., W-7851 Inzlingen (DE); Widmer, Ulrich, Dr., CH-4310 Rheinfelden (CH)
(74) Vertreter: Bertschinger, Christoph, Dr.

(56) Entgegenhaltungen:
- EP-A- 0 183 994

## Beschreibung

Die vorliegende Erfindung betrifft die Verbindung (S)-1-[(10-Chlor-6,7-dihydro-4-oxo-3-phenyl-4H-benzo[a]chino- lizin-1-yl)carbonyl]-3-äthoxypyrrolidin der Formel l.

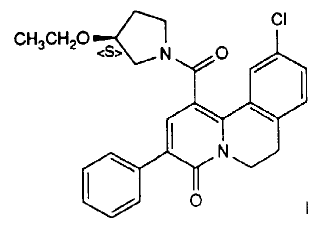

Die Verbindung der Formel 1 besitzt wertvolle pharmakologische Eigenschaften und kann zur Behandlung oder Prävention von Krankheiten verwendet werden. Sie besitzt insbesondere eine nichtsedierende, hypnotische, d.h. schlaffördernde, Wirkung und kann demnach zur Behandlung von Schlafstörungen verwendet werden.

Gegenstand der vorliegenden Erfindung ist die Verbindung der Formel l als solche und zur Anwendung als therapeutischen Wirkstoff: ein Verfahren zur Herstellung dieses Stoffes; diesen enthaltende Arzneimittel und deren Herstellung; die Verwendung dieses Stoffes bei der Behandlung oder Prävention von Krankheiten und zur Herstellung von Mitteln zur Behandlung von Schlafstörungen; sowie eine Methode zur Behandlung von Patienten, welche unter Schlafstörungen leiden.

Das der Verbindung der Formel l entsprechende Racemat, dessen Herstellung und dessen antikonvulsive Eigenschaften werden in der Europäischen Patentpublikation Nr. 183,994 beschrieben. In dieser Publikation wird ebenfalls erwähnt, dass sie muskelrelaxierende, sedativ-hypnotische und anxiolytische Eigenschaften besitzen soll.

Die Verbindung der Formel 1 kann hergestellt werden, indem man die Carbonsäure der Formel 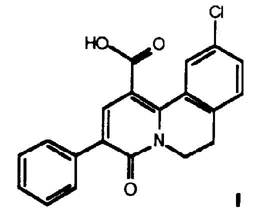 oder ein reaktives Derivat davon mit einem Amin der Formel 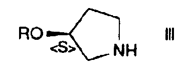 worin R Wasserstoff oder Aethyl bedeutet,
in das entsprechende Amid überführt und, sofern R Wasserstoff bedeutet, die erhaltene Verbindung der Formel 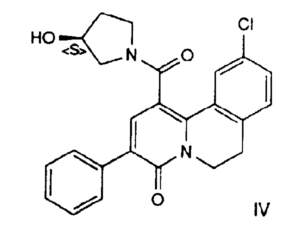 mit einem einen Aethylrest liefernden Mittel alkyliert.

Die Reaktion der freien Carbonsäure der Formel ll mit einem Amin der Formel lllwird vorzugsweise in Gegenwart eines Kondensationsmittels, wie O-Benzotriazol-1-yl-N,N,N',N'-tetramethyluronium-hexafluorophosphat oder N-Methyl-2-chlorpyridiniumjodid, in einem inerten organischen Lösungsmittel und in Gegenwart einer Base durchgeführt. Geeignete Lösungsmittel sind beispielsweise aromatische Kohlenwasserstoffe wie Benzol, Toluol und Xylol und N,N-Dimethylformamid. Geeignete Basen sind beispielsweise tertiäre Amine, wie Triäthylamin, 4-Methylmorpholin und dergleichen. Ein bevorzugtes Carbonsäurederivat, das in Gegenwart einer Base direkt mit dem Amin der Formel lll umgesetzt werden kann, ist das entsprechende Carbonsäurechlorid. Geeignete Basen sind wiederum die vorerwähnten tertiären Amine. Geeignete Lösungsmittel sind beispielsweise aromatische Kohlenwasserstoffe, wie Benzol, Toluol und Xylol, und Aether, wie Dioxan. Die Reaktion wird in beiden Fällen vorzugsweise in einem Bereich von Raumtemperatur bis Rückflusstemperatur des Reaktionsgemisches durchgeführt.

Bedeutet R im Amin der Formel lll Wasserstoff, so erhält man zunächst die Verbindung der Formel lV, welche anschliessend mit einem einen Aethylrest liefernden Mittel zur Verbindung der Formel 1 alkyliert wird. Diese Alkylierung wird zweckmässigerweise in einem inerten organischen Lösungsmittel, wie N,N-Dimethylformamid oder dergleichen, durchgeführt, wobei man als Base zweckmässigerweise eine starke Base, z.B. ein Alkalimetallhydrid oder -hydroxyd, wie Natriumhydrid, Kaliumhydroxyd und Natriumhydroxyd, verwendet. Man arbeitet zweckmässigerweise in einem Bereich von etwa 0°C bis Raumtemperatur. Als Alkylierungsmittel verwendet man vorzugsweise ein Aethylhalogenid, insbesondere Aethyljodid oder Aethylbromid, oder Diäthylsulfat.

Die Verbindung der Formel IV ist neu und ebenfalls Gegenstand der vorliegenden Erfindung.

Die als Ausgangsstoff verwendete Verbindung der Formel 111, worin R Wasserstoff bedeutet, ist eine bekannte Verbindung: vgl. Europäische Patentpublikation Nr. 304,087, Seite 11.

Die als Ausgangsstoff verwendete Verbindung der Formel 111, worin R Aethyl bedeutet, kann beispielsweise durch Alkylierung von (S)-1-Benzyl-3-pyrrolidinol mit einem Aethylhalogenid, wie Aethylbromid und Aethyljodid, in Gegenwart einer Base und anschliessendes Abspalten der Benzylgruppe durch katalytische Hydrogenolyse hergestellt werden. (S)-1-Benzyl-3-pyrrolidinol ist ebenfalls eine bekannte Verbindung; vgl. J. Med. Chem 29, 2504-2511 (1986) und Synth. Comm. 15, 587-598 (1985).

Die bisher verwendeten Schlafmittel, beispielsweise Barbiturate und Benzodiazepine, sind sedativ-hypnotisch wirksame Mittel. Diese Mittel wirken nicht nur hypnotisch, d.h. schlaffördernd, sondern auch sedativ. Ihre Verwendung führt daher zu einer generellen, unspezifischen Senkung der Vigilanz, die sich beispielsweise darin äussert, dass die kognitive, mnestische, reaktive, sensorische und motorische Leistungsfähigkeit auch im Wachzustand eingeschränkt ist. Dies führt unter Umständen zu gefährlichen Situationen in der Zeit zwischen Einnahme und Schlafeintritt sowie bei Unterbrechung des Schlafes zur Zeit ihrer vollen Wirksamkeit. Nichtsedierende Hypnotika sind dementsprechend Stoffe, die den Schlaf induzieren und erhalten, die Funktionen des Zentralnervensystems im Wachzustand aber nicht oder nur unwesentlich beeinträchtigen.

Es wurde nun überraschenderweise gefunden, dass die Verbindung der Formel 1 zwar hypnotisch, jedoch nicht sedativ wirksam ist, und daher die bekannten Nachteile der sedativ-hypnotischen Schlafmittel nicht aufweist.

Die schlafinduzierende Wirkung der Verbindung der Formel 1 kann im nachfolgend beschriebenen Versuch an Kaninchen gezeigt werden. Die Tiere werden unter Vollnarkose mit Elektroden in Hirngebieten versehen, deren verstärkte elektrische Signale (Elektroencephalogramm, EEG) die Unterscheidung von Wachsein (W), nicht-REM-Schlaf (NREMS) und REM-Schlaf (REMS) erlauben. REM-Schlaf ist Traumschlaf; er wird so genannt, weil darin schnelle Augenbewegungen Rapid Eye Movements) auftreten. Die Elektroden werden mit einem Stecker verbunden, der auf dem Schädel befestigt wird, so dass für den Versuch die Elektroden mit einem Kabel mit den Verstärkern und dem Aufzeichnungsgerät verbunden werden können. Nach Abschluss der Wundheilung werden die Tiere für zwei Tage in schallgedämpften Boxen untergebracht. An diesen zwei Tagen werden dann jeweils von 9 bis 15 Uhr die EEG's registriert (vgl. Scherschlicht und Marias in Brit. J. Clin. Pharmacol. 16, 295-355 [1983]). Am ersten Tag wird den Versuchstieren ein Vehikel peroral verabreicht (Kontrolle), am zweiten Tag peroral 0,1,0,3, 1 oder 10 mg/kg der Verbindung der Formel 1. Pro Dosis werden vier Tiere eingesetzt. Da das Kaninchen im Gegensatz zum Menschen nicht kontinuierlich schläft, wird die in NREMS und REMS verbrachte Zeit aufaddiert und pro Stunde in % von 60 Min. ausgedrückt. Die ermittelten Resultate sind in der nachfolgenden Tabelle zusammengestellt.

Die Resultate zeigen, dass die Verbindung der Formel 1 in allen eingesetzten Dosen die im NREMS verbrachte Zeit auf 70-75% der ersten Stunde der Schlafaufzeichnung steigert. Danach fällt die Menge an NREMS unterschiedlich schnell ab. Je höher die eingesetzte Dosis, desto länger hält die Wirkung an. Demgegenüber verbringen die Tiere an den Kontrolltagen etwa 50% jeder Stunde im NREMS. Der niedrigste Wert findet sich jeweils in der ersten Stunde der Aufzeichnung. Die Verbindung der Formel l zeigt keinen Einfluss auf den REMS.

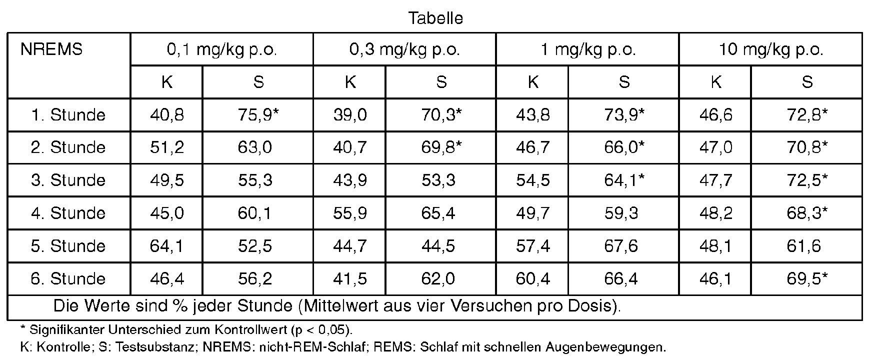

Dass die Verbindung der Formel 1 keine sedierende Wirkung hat, kann beispielsweise im Horizontal Wire Test (HWT) gezeigt werden. In diesem Test werden Mäuse oder Ratten so am Schwanz gehalten und aufgehoben, dass sie mit den Vorderpfoten einen horizontal gespannten Draht ergreifen können. Losgelassen ziehen sich normale Tiere sofort hoch und ergreifen den Draht auch mit den Hinterpfoten. Sedierend wirksame Substanzen bewirken, je nach Grad der Sedation, dass die Tiere bewegungslos hängen bleiben oder vom Draht herabfallen. Die Verbindung der Formel 1 wird den Versuchstieren peroral verabreicht und zeigte in Dosen bis zu 300 mg/kg weder an Mäusen noch an Ratten eine sedierende Wirkung. Die behandelten Versuchstiere verhalten sich wie unbehandelte, normale Tiere.

Die Verbindung der Formel 1 kann als Heilmittel, z.B. in Form von pharmazeutischen Präparaten, Verwendung finden. Die pharmazeutischen Präparate können peroral, z.B. in Form von Tabletten, Lacktabletten, Dragees, Hart-und Weichgelatinekapseln, Lösungen, Emulsionen oder Suspensionen, verabreicht werden Die Verabreichung kann aber auch rektal, z.B. in Form von Suppositorien, oder parenteral, z.B. in Form von Injektionslösungen, erfolgen.

Zur Herstellung von pharmazeutischen Präparaten kann das erfin dungsgemässe Produkt mit pharmazeutisch inerten, anorganischen oder organischen Trägern verarbeitet werden. Als solche Träger kann man für Tabletten, Lacktabletten, Dragees und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten Sie können auch noch andere therapeutisch wertvolle Stoffe enthalten. Arzneimittel enthaltend das erfindungsgemässe Produkt und einen therapeutisch inerten Träger, sowie ein Verfahren zu deren Herstellung, das dadurch gekennzeichnet ist, dass man das erfindungsgemässe Produkt und gegebenenfalls einen anderen therapeutisch wirksamen Stoff in eine galenische Darreichungsform bringt, sind ebenfalls Gegenstand der vorliegenden Erfindung.

Das erfindungsgemässe Produkt kann, wie bereits erwähnt, bei der Behandlung oder Prävention von Krankheiten, insbesondere bei der Behandlung von Schlafstörungen, sowie für die Herstellung von Arzneimitteln mit nichtsedierenden, hypnotischen Eigenschaften verwendet werden. Die Dosierung kann dabei innerhalb weiter Grenzen variieren und ist natürlich in jedem einzelnen Fall den individuellen Gegebenheiten anzupassen. Bei oraler Verabreichung liegt die wirksame Dosis in einem Bereich von etwa 0, 1 mg bis etwa 100 mg, vorzugsweise von etwa 0,5 mg bis etwa 20 mg.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

### Beispiel 1

a) 70,36 g 10-Chlor-6,7-dihydro-4-oxo-3-phenyl-4H-benzo[a]chinolizin-1-carbonsäure werden unter Argon in 1600 ml N,N-Dimethylformamid gelöst, worauf nacheinander 45 ml 4-Methylmorpholin, 27,2 g (S)-3-Hydroxypyrrolidin Hydrochlorid und 83,4 g O-Benzotriazol-1-yl-N,N,N',N'-tetramethyluronium-hexafluorophosphat zugegeben werden. Man rührt während etwa 18 Std. bei Raumtemperatur, giesst die erhaltene gelbe Lösung auf 6000 ml Wasser und versetzt langsam mit 2500 ml gesättigter Natriumhydrogencarbonatlösung. Das erhaltene Produkt wird abgesaugt und mit 1000 ml Wasser gewaschen. Nach Trocknen im Vakuum bei 70° erhält man 83,3 g Rohprodukt. Durch mehrmaliges Umkristallisieren aus der 120-fachen Menge Isopropanol und Chromatographieren der vereinigten Mutterlaugen an 1000 g Kieselgel mit Methylenchlorid/Diäthyläther (9:1) und anschliessend mit Methylenchlorid/Aceton (9:1) erhält man 74,2 g (88 %) (S)-1-[(10-Chlor-6,7-dihydro-4-oxo-3-phenyl-4H-benzo[a]chinolizin-1-yl)carbonyl]-3-hydroxypyrrolidin mit einem Smp. von 257-259°.
b) 64,3 g (S)-1-[(10-Chlor-6,7-dihydro-4-oxo-3-phenyl-4H-benzo[a]chinolizin-1-yl)carbonyl]-3-hydroxypyrrolidin werden in 900 ml N, N-Dimethylformamid durch leichtes Erwärmen (44°) gelöst. Man kühlt dann auf 13° ab, versetzt mit 31 ml Äthyljodid und kühlt auf 3-5° ab. Nach Zugabe von 17,1 g pulverisiertem Kaliumhydroxid wird bei etwa 0° während 5 Std gerührt. Die Reaktionsmischung wird dann auf 8000 ml Wasser gegossen und mit 50 ml 25-proz. Salzsäure angesäuert. Die Suspension wird über Nacht bei Raumtemperatur gerührt. Die Kristalle werden dann abgesaugt, mit Wasser gewaschen und bei 80° im Vakuum getrocknet. Durch Chromatographieren des erhaltenen Materials an 3000 g Kieselgel mit Methylenchlorid/Diäthyläther (9:1), (4:1), (3:1) und (2: 1) erhält man 58,5 g Rohprodukt und 4,4 g einer Mischung, welche aus Isopropanol umkristallisiert wird. Man erhält dabei 3,75 g eines nicht ganz reinen Produktes, das zusammen mit dem vorher erhaltenen Rohprodukt aus Isopropanol umkristallisiert wird. Man erhält insgesamt 54,7 g (80 %) (S)-1-[(10-Chlor-6,7-dihydro-4-oxo-3-phenyl-4H-benzo[a] chinolizin-1-yl)carbonyl]-3-äthoxypyrrolidin mit einem Smp. von 144-147°.

### Beispiel 2

a) 5 g Natriumhydrid (60%) werden in 100 ml trockenem Tetrahydrofuran suspendiert. Bei 0°C werden dann langsam 10 ml (S)-1-Benzyl-3-pyrrolidinol zugetropft. Nach beendeter Zugabe rührt man bei Raumtemperatur bis zum Ende der Wasserstoff-Entwicklung. Anschliessend wird noch eine Stunde weitergerührt. Es wird auf 0°C abgekühlt und dann langsam 9,75 ml Ethyljodid zugetropft. Man lässt auf Raumtemperatur erwärmen und rührt über Nacht weiter. Zur Zerstörung von überschüssigem Natriumhydrid tropft man unter Eiskühlung 50 ml Methanol hinzu. Das Reaktionsgemisch wird im Vakuum eingeengt und das verbleibende Oel in 200 ml Methylenchlorid aufgenommen und zweimal mit je 100 ml gesättigter Kochsalz-Lösung ausgeschüttelt. Die organische Phase wird über Natriumsulfat getrocknet und anschliessend im Vakuum eingeengt. Man erhält 14 g gelbes Oel. Das Rohprodukt wird an 140 g Kieselgel mit Hexan/Essigsäureethylester (2:1) chromatographiert. Man erhält 9,3 g (75%) (S)-1-Benzyl-3-ethoxypyrrolidin als gelbliches Oel.
b) In 100 ml Methanol werden 10 g (S)-1-Benzyl-3-ethoxypyrrolidin gelöst, mit 1 g 10% Palladium auf Kohle versetzt und unter einer Wasserstoff-Atmosphäre gerührt. Nach 4 Stunden erfolgt keine Wasserstoffaufnahme mehr. Es wird vom Katalysator über ein Dicalite-Polster abfiltriert und der Rückstand im Vakuum eingeengt. Man erhält 5,3 g (95%) (S)-3-Ethoxypyrrolidin als gelbes Oel.
c) 7,04 g 10-Chlor-6,7-dihydro-4-oxo-3-phenyl-4H-benzo[a]chinolizin-1-carbonsäure werden unter Argon in 100 ml Essigsäureethylester suspendiert und mit 2,1 ml Oxalylchlorid versetzt. Anschliessend werden 0,2 ml N,N-Dimethylformamid zugesetzt, wobei eine Gasentwicklung beobachtet wird. Man lässt 30 Minuten bei Raumtemperatur rühren und setzt nochmals 0,2 ml Oxalylchlorid und darauf 0,1 ml N,N-Dimethylformamid zu und lässt weitere 30 Minuten bei Raumtemperatur rühren. Das Reaktionsgemisch wird im Eisbad auf 0-5°C abgekühlt, wobei Kristalle ausfallen. Es werden 13,3 ml Triethylamin zugesetzt und anschliessend eine Lösung von 2,53 g (S)-3-Ethoxypyrrolidin in 25 ml Essigsäureethylester zugetropft. Anschliessend wird 30 Minuten bei ca. 0°C gerührt.

Die Reaktionsmischung wird zweimal mit je 50 ml Wasser gewaschen und die vereinigten Wasserephasen einmal mit 20 ml Essigsäureethylester extrahiert. Die vereinigten organischen Phasen werden über Natriumsulfat getrocknet, filtriert und eingedampft, wobei 9,0 g Rohprodukt erhalten werden. Das Rohprodukt wird in 50 ml Methylenchlorid gelöst und durch 45 g Kieselgel filtriert (Methylenchlorid/Aceton 9:1), wobei 8,6 g gelbe Kristalle erhalten werden. Diese werden in 100 ml tert.-Butylmethylether aufgenommen und während 1 Stunde zum Rückfluss erhitzt. Man lässt auf Raumtemperatur abkühlen und saugt die Kristalle (6,95 g) ab. 5,5 g des so erhaltenen Rohproduktes werden aus 55 ml Essigsäureethylester umkristallisiert, wobei zur Kristallisation im Eisbad gekühlt wird.

Man erhält nach Trocknung bei 80°C/0,05 mm (18 Std.) 4,2 g (S)-1-[(10-Chior-6,7-dihydro-4-oxo-3-phenyi-4H-benzo[a]chinolizin-1-yl)carbonyl]-3-ethoxypyrrolidin vom Smp 134-136°C.

### Beispiel A

Die Verbindung der Formel kann in an sich bekannter Weise als Wirkstoff zur Herstellung von pharmazeutischen Präparaten nachfolgender Zusammensetzung verwendet werden:

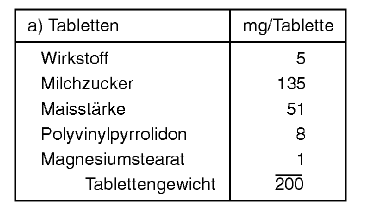

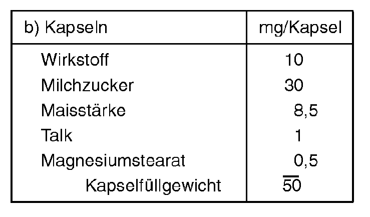

## Patentansprüche (Patentansprüche für folgende(n) Vertragsstaat(en) : AT, BE, CH, DE, DK, FR, GB, IT, LI, LU, MC, NL, PT, SE)

1. Die Verbindung (S)-1-[(10-Chlor-6,7-dihydro-4-oxo-3-phenyl-4H-benzo[a]chinolizin-1-yl)carbonyl]-3-äthoxypyrro- lidin der Formel

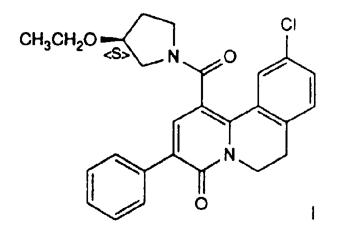

2. (S)-1-[(10-Chlor-6,7-dihydro-4-oxo-3-phenyl-4H-benzo[a]chinolizin-1-yl)carbonyl]-3-hydroxypyrrolidin

3. Die Verbindung nach Anspruch 1 zur Anwendung als therapeutischer Wirkstoff.

4. Die Verbindung nach Anspruch 1 zur Anwendung als nichtsedierender, hypnotisch wirksamer Stoff.

5. Verfahren zur Herstellung der Verbindung nach Anspruch 1, dadurch gekennzeichnet, dass man die Carbonsäure der Formel 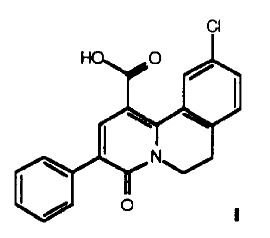 oder ein reaktives Derivat davon mit einem Amin der Formel 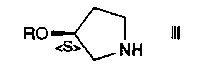 worin R Wasserstoff oder Aethyl bedeutet,
in das entsprechende Amid überführt und, sofern R Wasserstoff bedeutet, die erhaltene Verbindung der Formel 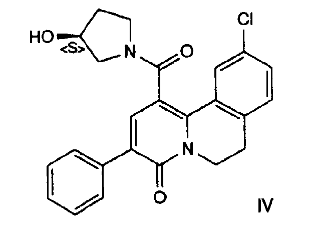 mit einem einen Aethylrest liefernden Mittel alkyliert.

6. Arzneimittel, enthaltend die Verbindung nach Anspruch 1 und ein therapeutisch inertes Trägermaterial.

7. Mittel zur Behandlung von Schlafstörungen, insbesondere nichtsedierende Hypnotika, enthaltend die Verbindung nach Anspruch 1 und ein therapeutisch inertes Trägermaterial.

8. Verwendung der Verbindung nach Anspruch 1 zur Herstellung von Mitteln zur Behandlung von Schlafstörungen, insbesondere von nichtsedierenden Hypnotika.

## Patentansprüche (Patentansprüche für folgende(n) Vertragsstaat(en) : ES)

1. Verfahren zur Herstellung der Verbindung (S)-1-[(10-Chlor-6,7-dihydro-4-oxo-3-phenyl-4H-benzo[a]chinolizin-1-yl)carbonyl]-3-äthoxypyrrolidin der Formel 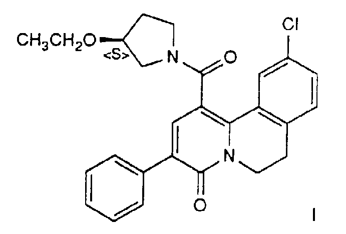 dadurch gekennzeichnet, dass man die Carbonsäure der Formel 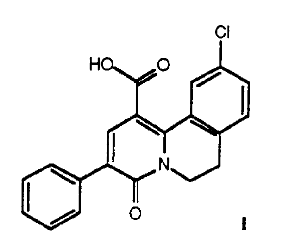 oder ein reaktives Derivat davon mit einem Amin der Formel 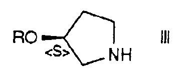 worin R Wasserstoff oder Aethyl bedeutet,
in das entsprechende Amid überführt und, sofern R Wasserstoff bedeutet, die erhaltene Verbindung der Formel 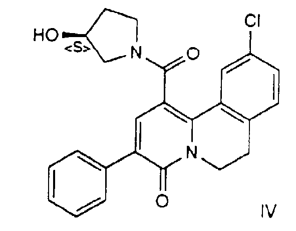 mit einem einen Aethylrest liefernden Mittel alkyliert.

2. Verfahren zur Herstellung von Mitteln, insbesondere zur Behandlung von Schlafstörungen, dadurch gekennzeichnet, dass man die Verbindung der in Anspruch 1 angegebenen Formel und gegebenenfalls einen anderen therapeutisch wirksamen Stoff zusammen mit einem therapeutisch inerten Trägermaterial in eine galenische Darreichungsform bringt.

3. Verwendung der Verbindung der in Anspruch 1 angegebenen Formel I zur Herstellung von Mitteln zur Behandlung von Schlafstörungen, insbesondere von nichtsedierenden Hypnotika.

## Patentansprüche (Patentansprüche für folgende(n) Vertragsstaat(en) : GR)

1. Verfahren zur Herstellung der Verbindung (S)-1-[(10-Chlor-6,7-dihydro-4-oxo-3-phenyl-4H-benzo[a]chinolizin-1-yl)carbonyl]-3-äthoxypyrrolidin der Formel 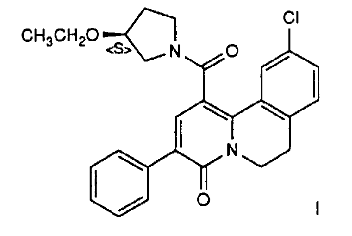 dadurch gekennzeichnet, dass man die Carbonsäure der Formel 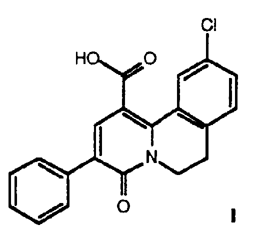 oder ein reaktives Derivat davon mit einem Amin der Formel 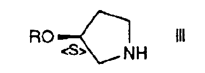 worin R Wasserstoff oder Aethyl bedeutet,
in das entsprechende Amid überführt und, sofern R Wasserstoff bedeutet, die erhaltene Verbindung der Formel 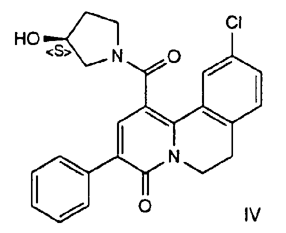 mit einem einen Aethylrest liefernden Mittel alkyliert.

2. Verfahren zur Herstellung von Mitteln, insbesondere zur Behandlung von Schlafstörungen, dadurch gekennzeichnet, dass man die Verbindung der in Anspruch 1 angegebenen Formel und gegebenenfalls einen anderen therapeutisch wirksamen Stoff zusammen mit einem therapeutisch inerten Trägermaterial in eine galenische Darreichungsform bringt.

3. Verwendung der Verbindung der in Anspruch 1 angegebenen Formel I zur Herstellung von Mitteln zur Behandlung von Schlafstörungen, insbesondere von nichtsedierenden Hypnotika.

4. (S)-1-[(10-Chlor-6,7-dihydro-4-oxo-3-phenyl-4H-benzo[a]chinolizin-1-yl)carbonyl]-3-hydroxypyrrolidin

## Claims (Claims for the following Contracting State(s) : AT, BE, CH, DE, DK, FR, GB, IT, LI, LU, MC, NL, PT, SE)

1. The compound (S)-1-[(10-chloro-6,7-dihydro-4-oxo-3-phenyl-4H-benzo[a]quinolizin-1-yl)carbonyl)-3-ethoxypyr- rolidine of the formula

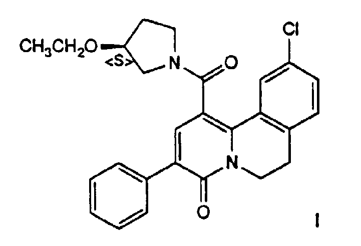

2. (S)-1-[(10-Chloro-6,7-dihydro-4-oxo-3-phenyl-4H-benzo[a]quinolizin-1-yl)carbonyl]-3-hydroxyprrolidine.

3. The compound according to claim 1 for use as a therapeutically active substance.

4. The compound according to claim 1 for use as a non-sedating, hypnotically active substance.

5. A process for the manufacture of the compound according to claim 1, which process comprises converting the carboxylic acid of the formula 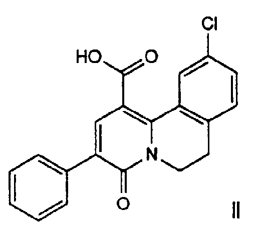 or a reactive derivative thereof into the corresponding amide with an amine of the formula 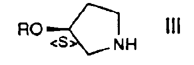 wherein R signifies hydrogen or ethyl,
and, when R signifies hydrogen, alkylating the resulting compound of the formula 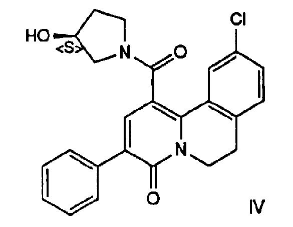 with an agent yielding the ethyl group.

6. A medicament containing the compound according to claim 1 and a therapeutically inert carrier material.

7. A medicament for the treatment of sleep disorders, especially an non-sedating hypnotic, containing the compound according to claim 1 and a therapeutically inert carrier material.

8. The use of the compound according to claim 1 for the manufacture of medicaments for the treatment of sleep disorders, especially of non-sedating hypnotics.

## Claims (Claims for the following Contracting State(s) : ES)

1. A process for the manufacture of the compound (S)-1-[(10-chloro-6,7-dihydro-4-oxo-3-phenyl-4H-benzo[a]quino- lizin-1-yl)carbonyl)-3-ethoxypyrrolidine of the formula 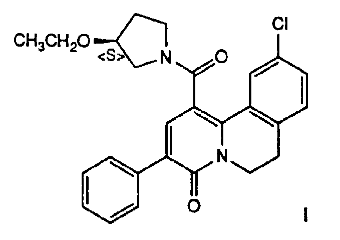 which process comprises converting the carboxylic acid of the formula 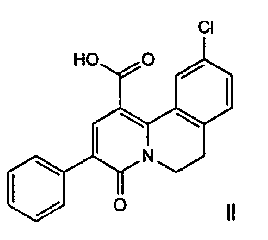 or a reactive derivative thereof into the corresponding amide with an amine of the formula 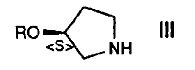 wherein R signifies hydrogen or ethyl,
and, when R signifies hydrogen, alkylating the resulting compound of the formula 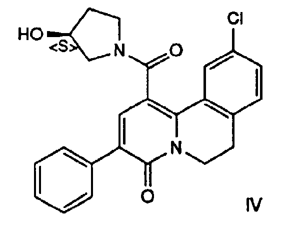 with an agent yielding the ethyl group.

2. A process for the manufacture of agents, particularly for the treatment of sleep disorders, characterized in that the compound of formula I according to claim 1 and, optionally, another therapeutically active compound, together with a therapeutically inert carrier material is braught into a galenical application form.

3. The use of the compound according to claim 1 for the manufacture of medicaments for the treatment of sleep disorders, especially of non-sedating hypnotics.

## Claims (Claims for the following Contracting State(s) : GR)

1. A process for the manufacture of the compound (S)-1-[(10-chloro-6,7-dihydro-4-oxo-3-phenyl-4H-benzo[a]quino- lizin-1-yl)carbonyl}-3-ethoxypyrrolidine of the formula 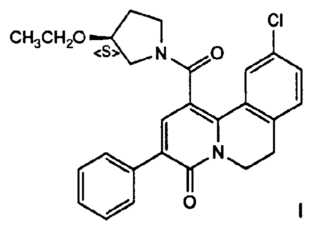 which process comprises converting the carboxylic acid of the formula 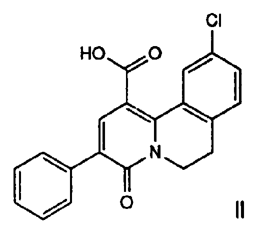 or a reactive derivative thereof into the corresponding amide with an amine of the formula 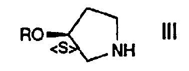 wherein R signifies hydrogen or ethyl,
and, when R signifies hydrogen, alkylating the resulting compound of the formula 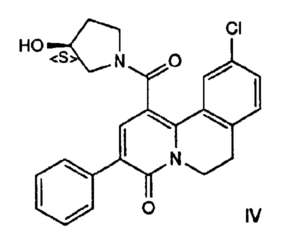 with an agent yielding the ethyl group.

2. A process for the manufacture of agents, particularly for the treatment of sleep disorders, characterized in that the compound of formula I according to claim 1 and, optionally, another therapeutically active compound, together with a therapeutically inert carrier material is braught into a galenical application form.

3. The use of the compound according to claim 1 for the manufacture of medicaments for the treatment of sleep disorders, especially of non-sedating hypnotics.

4. (S)-1-[(10-Chloro-6,7-dihydro-4-oxo-3-phenyl-4H-benzo[a]quinolizin-1-yl)carbonyl]-3-hydroxyprrolidine.

## Revendications (Revendications pour l'(les) Etat(s) contractant(s) suivant(s) : AT, BE, CH, LI, DE, DK, FR, GB, IT, LU, MC, NL, PT, SE)

1. Le composé : (S)-1-[(10-chloro-6,7-dihydro-4-oxo-3-phényl-4H-benzo[a]quinolizine-1-yl)-carbonyl]-3-éthoxypyr- rolidine de formule

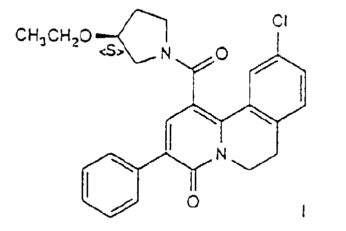

2. La (S)-1-[(10-chloro-6,7-dihydro-4-oxo-3-phényl-4H-benzo[a]quinolizine-1-yl)-carbonyl]-3-hydroxypyrrolidine.

3. Le composé selon revendication 1, pour l'utilisation en tant que substance active thérapeutique.

4. Le composé selon revendication 1, pour l'utilisation en tant que substance à activité hypnotique non sédative.

5. Procédé de préparation du composé selon revendication 1, caractérisé en ce que l'on convertit l'acide carboxylique de formule 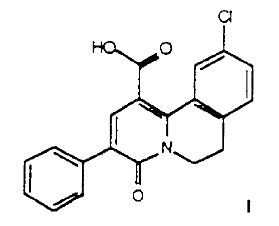 ou l'un de ses dérivés réactifs, par réaction avec une amine de formule 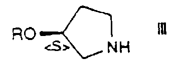 dans laquelle R représente l'hydrogène ou un groupe éthyle,
en l'amide correspondant et, lorsque R représente l'hydrogène, on alkyle le composé obtenu, de formule 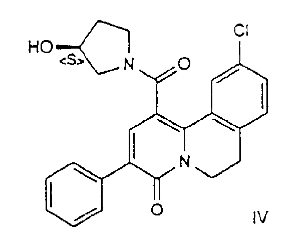 à l'aide d'un agent apportant un groupe éthyle.

6. Médicament contenant le composé selon revendication 1 et un véhicule inerte du point de vue thérapeutique.

7. Agents pour le traitement des troubles du sommeil, en particulier agents hypnotiques non sédatifs, contenant le composé selon revendication 1 et un véhicule inerte du point de vue thérapeutique.

8. Utilisation du composé selon revendication 1 pour la préparation d'agents pour le traitement des troubles du sommeil, en particulier d'agents hypnotiques non sédatifs.

## Revendications (Revendications pour l'(les) Etat(s) contractant(s) suivant(s) : ES)

1. Procédé de préparation du composé : (S)-1-[(10-chloro-6,7-dihydro-4-oxo-3-phényl-4H-benzo[a]-quinolizine-1-yl)-carbonyl]-3-éthoxypyrrolidine de formule 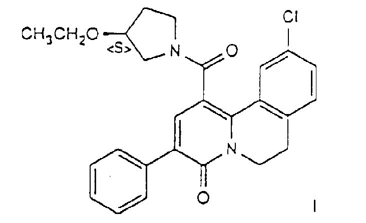 caractérisé en ce que l'on convertit l'acide carboxylique de formule 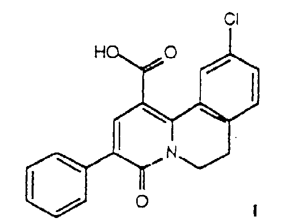 ou l'un de ses dérivés réactifs, par réaction avec une amine de formule 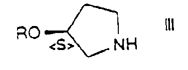 dans laquelle R représente l'hydrogène ou un groupe éthyle,
en l'amide correspondant et, lorsque R représente l'hydrogène, on alkyle le composé obtenu de formule 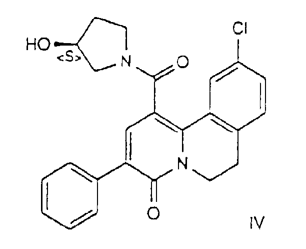 à l'aide d'un réactif apportant un groupe éthyle.

2. Procédé de préparation d'agents servant en particulier au traitement des troubles du sommeil, caractérisé en ce que l'on met le composé de formule 1 selon revendication 1, et le cas échéant une autre substance possédant une activité thérapeutique sous une forme d'administration galénique avec un véhicule inerte du point de vue thérapeutique.

3. Utilisation du composé de formule I selon revendication 1 pour la préparation d'agents servant au traitement des troubles du sommeil, en particulier d'agents hypnotiques non sédatifs.

## Revendications (Revendications pour l'(les) Etat(s) contractant(s) suivant(s) : GR)

1. Procédé de préparation du composé : (S)-1-[(10-chloro-6,7-dihydro-4-oxo-3-phényl-4H-benzo[a]-quinolizine-1-yl)-carbonyl]-3-éthoxypyrrolidine de formule 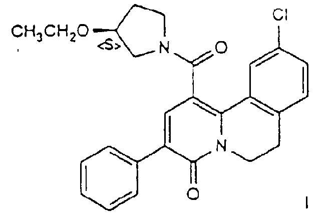 caractérisé en ce que l'on convertit l'acide carboxylique de formule 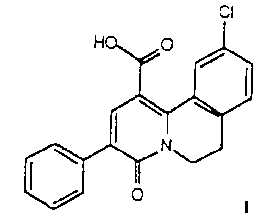 ou l'un de ses dérivés réactifs, par réaction avec une amine de formule 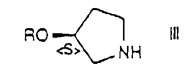 dans laquelle R, représente l'hydrogène ou un groupe éthyle, en l'amide correspondant et, lorsque R représente l'hydrogène, on alkyle le composé obtenu de formule 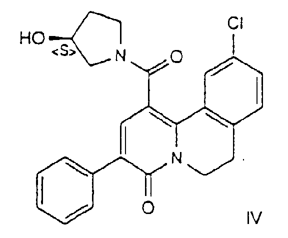 à l'aide d'un réactif apportant un groupe éthyle.

2. Procédé de préparation d'agents servant en particulier au traitement des troubles du sommeil, caractérisé en ce que l'on met le composé de formule 1 selon revendication 1, et le cas échéant une autre substance possédant une activité thérapeutique sous une forme d'administration galénique avec un véhicule inerte du point de vue thérapeutique.

3. Utilisation du composé de formule I selon revendication 1 pour la préparation d'agents servant au traitement des troubles du sommeil, en particulier d'agents hypnotiques non sédatifs.

4. La (S)-1-[(10-chloro-6,7-dihydro-4-oxo-3-phényl-4H-benzo[a]quinolizine-1-yl-carbonyl]-3-hydroxypyrrolidine.
